(19) Europäisches Patentamt

European Patent Office

Office européen des brevets

(11) Veröffentlichungsnummer: **0 209 659**
**A2**

(12) **EUROPÄISCHE PATENTANMELDUNG**

(21) Anmeldenummer: 86106590.2

(22) Anmeldetag: 15.05.86

(51) Int. Cl.⁴: **A61M 5/16**

(30) Priorität: 16.07.85 DE 3525264

(43) Veröffentlichungstag der Anmeldung:
28.01.87 Patentblatt 87/05

(84) Benannte Vertragsstaaten:
AT BE CH DE FR GB IT LI LU NL SE

(71) Anmelder: **INTERMEDICAT GMBH**
**Gerliswilstrasse 74**
**CH-6020 Emmenbrücke(CH)**

(72) Erfinder: **Knoche, Alfred, Dipl.-Ing.**
**Herkulesstrasse 8**
**D-3501 Körle(DE)**
Erfinder: **Proell, Dieter**
**An der Laube 10**
**D-3501 Körle(DE)**
Erfinder: **Hessberg, Sigfried, Dipl.-Ing.**
**Im Nick 29**
**D-3508 Melsungen(DE)**
Erfinder: **Heiget, Rudolf**
**Zum Roten Rain 21**
**D-3508 Melsungen 5(DE)**
Erfinder: **May, Karl-Georg**
**Pfieffrain 48**
**D-3508 Melsungen(DE)**

(74) Vertreter: **von Kreisler, Alek, Dipl.-Chem. et al**
**Deichmannhaus am Hauptbahnhof**
**D-5000 Köln 1(DE)**

(54) Tropfendetektor.

㊼ Ein Lichtsender liefert parallele Lichtstrahlen (22), die quer durch eine lichtdurchlässige Tropfkammer (13) hindurchgehen. Hinter der Tropfkammer (13) ist ein Lichtstrahl-Querschnittswandler (23) angeordnet, der die gesamte Breite der Tropfkammer erfaßt und die auf seiner Eintrittsseite (25) einfallenden Strahlen (22) vollflächig auf einen Lichtempfänger (27) leitet. Der Lichtstrahl-Querschnittswandler (23) enthält Lichleiter (24), deren Enden (24a) auf der Eintrittsseite (25) parallel zueinander verlaufen und deren andere Enden (24b) auf der Austrittsseite (26) ebenfalls parallel zueinander verlaufen. Die Enden (24a) an der Eintrittsseite (25) sind entlang eines Streifens angeordnet, während die Enden (24b) auf der Austrittsseite (26) in Form einer Kreisscheibe angeordnet sind, die in Form und Größe der Sensorfläche des Lichtempfängers (27) entspricht.

FIG. 3

## Tropfendetektor

Die Erfindung betrifft einen Tropfendetektor mit einem Lichtsender und einem Lichtempfänger, die an entgegengesetzten Seiten einer lichtdurchlässigen Tropfkammer positionierbar sind.

In der Medizintechnik ist es bekannt, die einem Patienten zuzuführende Menge einer Infusionsflüssigkeit durch Tropfenzählung zu überwachen und zu regeln. Zu diesem Zweck ist zwischen dem die Infusionslösung enthaltenden Behälter und einer Pumpe oder einem Regelmechanismus, die in dem Schlauchsystem zwischen dem Behälter und dem Patienten angeordnet ist, eine Tropfkammer vorgesehen, in die die Infusionslösung hineintropft. An der Tropfkammer befindet sich ein Tropfendetektor, der das Fallen der einzelnen Tropfen erkennt und einen Tropfenzähler steuert. Der Tropfenzähler steuert die Pumpe bzw. Regelmechanismus in der Weise, daß in einer bestimmten Zeiteinheit eine bestimmte Tropfenzahl den Behälter verläßt und dem Patienten zugeführt wird. Der Tropfendetektor ist nach Art einer Lichtschranke ausgebildet, in deren Strahlungsweg sich die Tropfkammer befindet. Der Lichtsender und der Lichtempfänger sind in einem Gehäuse angebracht, das die Tropfkammer umschließt, wobei das Eintreten von Fremdlicht in den Strahlenweg zwischen Lichtsender und Lichtempfänger vermieden werden muß.

Der die Infusionslösung enthaltende Behälter wird mit nach unten weisender Öffnung, die durch einen elastomeren Stopfen verschlossen ist, aufgehängt. Der Stopfen wird mit einem an der Tropfkammer befindlichen Einstichdorn, der einen längslaufenden Kanal aufweist, durchstochen. Da der Behälter mit an seinem unteren Ende befindlicher Tropfkammer herabhängt, kann es vorkommen, daß Behälter und Tropfkammer eine Schräglage einnehmen. Die Tropfen fallen dann nicht entlang der Achse der Tropfkammer herab, sondern sie treffen auf die Seitenwand. Damit das durch die Tropfkammer quer hindurchgehende Strahlenbündel des Tropfendetektors auch bei Schräglage die herabfallenden Tropfen erfaßt, muß dieses Strahlenbündel, ebenso wie der Erfassungsbereich des Lichtempfängers, einen relativ großen Durchmesser haben. Bei einem großen Durchmesser dieses Strahlenbündels und des Erfassungsbereichs besteht aber die Gefahr, daß auch Rückspritzer, die entstehen, wenn ein Tropfen auf die im unteren Teil der Tropfkammer enthaltene Flüssigkeit trifft, in den Erfassungsbereich gelangen und als Tropfen registriert werden.

Bei den bekannten Tropfendetektoren, bei denen der Lichtempfänger eine kreisförmige lichtempfindliche Sensorfläche aufweist, hat der Erfassungsbereich, wenn eine großflächige Ausleuchtung der Tropfkammer erfolgt, in Längsrichtung (Achsrichtung) der Tropfkammer die gleiche Größe wie in Querrichtung. Dies bedeutet, daß der Erfassungsbereich sehr nahe an den Flüssigkeitsstand in der Tropfkammer heranreicht, so daß auch Rückspritzer erfaßt werden. Wenn andererseits nur ein schmaler Streifen der Tropfkammer ausgeleuchtet wird, entspricht die Abbildung dieses Streifens nur einem geringen Teil der Sensorfläche und des Lichtempfängers, so daß die Signalausbeute gering ist. Ein weiterer Nachteil besteht darin, daß Tropfen, die in der Nähe des Randes des Erfassungsbereiches erkannt werden, ein geringeres Signal liefern als Tropfen, die in der Mitte des Erfassungsbereichs fallen.

Der Erfindung liegt die Aufgabe zugrunde, einen Tropfendetektor der eingangs genannten Art zu schaffen, bei dem die Signalausbeute verbessert ist und bei dem fallende Tropfen mit größerer Sicherheit erkannt werden.

Zur Lösung dieser Aufgabe ist erfindungsgemäß vorgesehen, daß im Strahlenweg vor dem Lichtempfänger ein Lichtstrahl-Querschnittswandler angeordnet ist, der einen der im wesentlichen der lichten Weite der Tropfkammer entsprechenden Erfassungsbereich aufweist und die aus dem Erfassungsbereich einfallende Strahlung in ein Strahlenbündel umsetzt, das die lichtempfindliche Sensorfläche des Lichtempfängers im wesentlichen ausfüllt.

Bei dem erfindungsgemäßen Tropfenempfänger wird der Querschnitt des Erfassungsbereichs, in dem Tropfen erkannt werden, auf die Form und/oder Größe der Sensorfläche umgesetzt. Solange kein fallender Tropfen vorhanden ist, trifft das Licht aus dem gesamten Erfassungsbereich auf die Sensorfläche, die somit voll beleuchtet ist. Passiert ein Tropfen irgendeine Stelle des Erfassungsbereichs, der durch den Lichtstrahl-Querschnittswandler vorgegeben ist, dann unterbricht dieser Tropfen den Strahlenweg, so daß auf die Sensorfläche weniger Licht fällt. Dadurch daß der gesamte Flächenbereich der Sensorfläche aus dem Erfassungsbereich beleuchtet wird, ergibt sich eine hohe Empfindlichkeit des Lichtempfängers und eine hohe Sicherheit der Tropfenerkennung. An der Sensorfläche sind keine Flächenbereiche, die ständig verdunkelt sind, so daß eine vorübergehende Verdunkelung eines Teils der Sensorfläche am Lichtempfänger eine relativ große Potentialänderung gegenüber dem Zustand ohne Verdunkelung bewirkt.

Der Lichtstrahl-Querschnittswandler setzt die Fläche des Erfassungsbereichs um in eine Fläche, die in Form und Größe der Sensorfläche entspricht. Der Erfassungsbereich des Lichtstrahl-Querschnittswandlers wird von dem Lichtsender vollflächig ausgeleuchtet.

Gemäß einer bevorzugten Ausführungsform der Erfindung ist vorgesehen, daß der Lichtstrahl-Querschnittswandler aus einem Bündel von Lichtleitern besteht, dessen Querschnittsform an der Eintrittsseite von der Querschnittsform an der Austrittsseite abweicht. Mit einfachen Mitteln wird hierdurch die Form und/oder Größe des Erfassungsbereichs in die Form und /oder Größe der Sensorfläche umgesetzt. Wenn der Erfassungsbereich die gleiche Form hat wie die Sensorfläche, muß lediglich eine Größenumwandlung erfolgen. Hat der Erfassungsbereich dagegen eine andere Form als die Sensorfläche, kann durch entsprechende Verlegung der Lichtleiter in dem Bündel erreicht werden, daß z.B. ein rechteckiger oder ellipsenförmiger Erfassungsbereich in einen z.B. kreisförmigen Bereich, der der kreisförmigen Sensorfläche entspricht, umgesetzt wird.

Lichtempfindliche Halbleiter liefern ihr maximales elektrisches Ausgangssignal, wenn das Licht senkrecht auf sie auftrifft. Bei einem Einfallswinkel von 45° ist das elektrische Signal bereits sehr klein. Damit die Strahlung aus dem Erfassungsbereich senkrecht auf die Sensorfläche des Lichtempfängers trifft, ist gemäß einer bevorzugten Weiterbildung der Erfindung vorgesehen, daß die Lichtleiter an der Eintrittsseite des Bündels parallel zueinander verlaufen. Ferner ist vorgesehen, daß die Enden der Lichtleiter an der Austrittsseite des Bündels parallel zueinander verlaufen. Auf diese Weise wird erreicht, daß ein Tropfen, der in der Nähe der Wand der Tropfkammer fällt, am Lichtempfänger die gleiche Signalausbeute liefert wie ein Tropfen der in der Mitte der Tropfkammer fällt.

Zweckmäßigerweise hat der Erfassungsbereich die Form einer Scheibe, die quer durch die Tropfkammer hindurchgeht und rechtwinklig zur Tropfkammerachse verläuft und deren Dicke wesentlich kleiner ist, als ihr Durchmesser. Ein solcher -scheibenförmiger Erfassungsbereich hat den Vorteil, daß er eine geringe Erstreckung in Längsrichtung der Tropfkammerachse hat, so daß Rückspritzer nicht in ihn eindringen können. Der Erfassungsbereich des Lichtstrahl-Querschnittswandlers und somit des Lichtempfängers hat eine geringe vertikale Höhe und er ist -in Seitenansicht der Tropfkammer -streifenförmig. Der Inhalt dieses Streifens wird vollflächig auf die Sensorfläche übertragen, d.h. jedem Punkt des Streifens entspricht irgendein Punkt der Sensorfläche. Sofern zwischen Tropfkammer und Lichtempfänger kein vergrößerndes oder verkleinerndes optisches System angeordnet ist, entspricht der Flächeninhalt des (rechteckigen) Streifens demjenigen der (z.B. runden) Sensorfläche.

Im folgenden werden unter Bezugnahme auf die Zeichnun gen Ausführungsbeispiele der Erfindung näher erläutert.

Es zeigen:

Fig. 1 eine schematische Darstellung eines Infusionssystems unter Verwendung des Tropfendetektors,

Fig. 2 eine schematische Darstellung einer ersten Ausführungsform des Tropfendetektors,

Fig. 3 eine genauere Darstellung des Lichtstrahl-Querschnittswandlers aus Fig. 2,

Fig. 4 eine Ansicht des Lichtstrahl-Querschnittswandlers aus Richtung der Linie VI-VI in Fig. 3,

Fig. 5 eine Ansicht aus Richtung der Linie V-V in Fig. 3,

Fig. 6 in gleicher Darstellung wie Fig. 2 eine andere Ausführungsform des Tropfendetektor,

Fig. 7 eine Ansicht des Tropfendetektors, der an einer Tropfkammer befestigt ist, deren Einstichdorn durch den Stopfen einer eine Infusionslösung enthaltenen Flasche hindurchgestochen ist,

Fig. 8 eine Draufsicht des Tropfendetektors von oben, also bei geöffnetem Deckel, aus Richtung der Linie VIII-VIII von Fig. 7,

Fig. 9 eine Seitenansicht des Tropfendetektors aus Richtung des Pfeiles IX in Fig. 7 und

Fig. 10 einen Schnitt entlang der Linie X-X von Fig. 9.

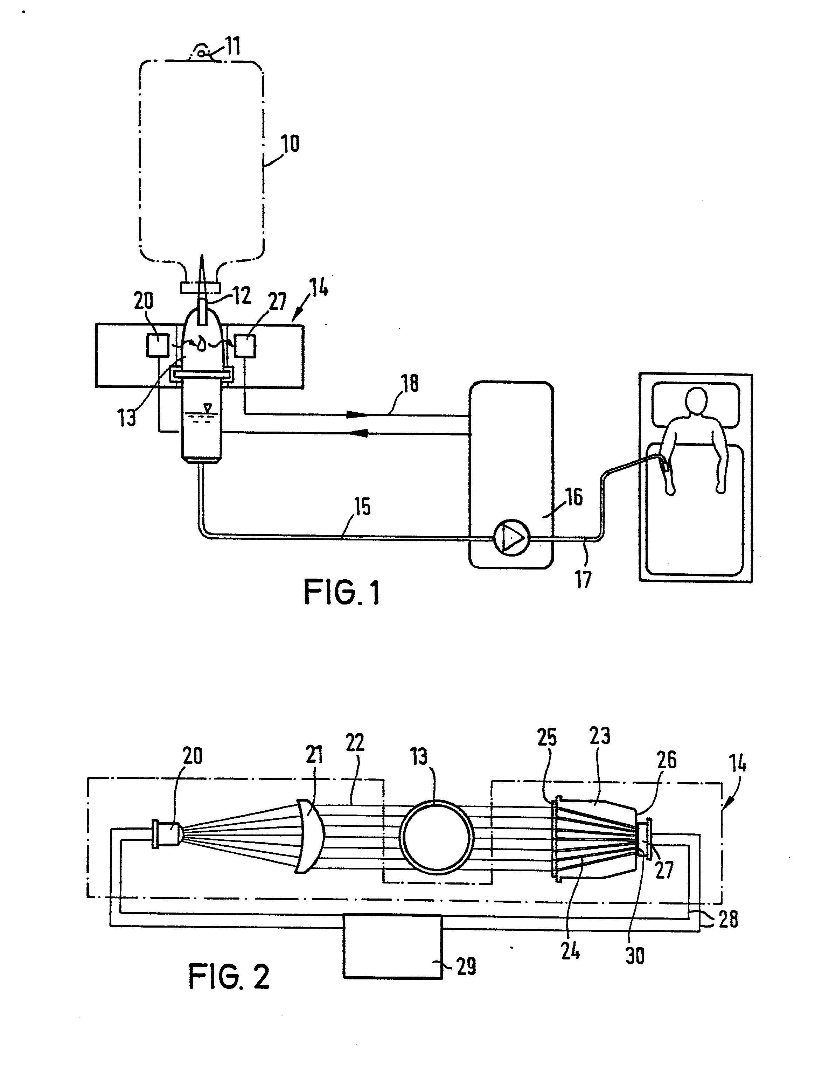

Bei dem in Fig. 1 dargestellten Infusionssystem ist ein Behälter 10 vorgesehen, der mit nach unten weisender Öffnung an einem (nicht dargestellten) Gestell aufgehängt ist. Zu diesem Zweck ist am Behälterboden eine Aufhängeöse 11 angeordnet. Die Behälteröffnung ist mit einem elastischen Stopfen verschlossen, durch den der Einstichdorn 12, der Tropfkammer 13 hindurchgestochen ist. Durch die Reibung zwischen Einstichdorn 12 und Stopfen wird die Tropfkammer 13 an dem Behälter 10 festgehalten: An der Tropfkammer 13 ist der Tropfendetektor 14 befestigt. Vom unteren Ende der Tropfkammer 13 führt ein Schlauch 15 zu der Pumpe 16 oder einem Regler und von dieser führt ein weiterer Schlauch 17 zum Patienten. Die elektronischen Signale werden durch den Regler bzw. die Pumpe ausgewertet. Die Regelung der Pumpe 16 erfolgt über eine elektrische Leitung 18 in Abhängigkeit von den elektrischen Signalen des Tropfendetektors 14.

Die Tropfkammer 13 weist eine runde Wand aus lichtdurchlässigem Material auf. An dieser Wand ist der Tropfendetektor 14 so befestigt, daß die Lichtstrahlen quer durch denjenigen Bereich der Tropfkammer 13 hindurchgehen, indem die Tropfen fallen (Fig. 2). Der Tropfendetektor weist einen Lichtsender 20, beispielsweise eine lichtemittierende Diode auf. Im Strahlenweg hinter dem Lichtsender 20 ist eine Sammellinse 21 angeordnet, die das Licht in ein paralleles Strahlenbündel 22 umwandelt, das durch die Tropfkammer 13 quer zu deren Längsachse hindurchgeht und die gesamte Breite der Tropfkammer erfaßt. Im Strahlenweg hinter der Tropfkammer 13 ist der Lichtstrahl-Querschnittswandler 23 angeordnet, der bei dem vorliegenden Ausführungsbeispiel aus einem Bündel aus Lichtleiterfasern 24 besteht. Auf der Eintrittsseite 25 sind die Enden der Lichtleiter 24 entlang eines horizontalen Streifens angeordnet und auf der Austrittsseite 26 sind die Enden der Lichtleiter in Form einer Kreisscheibe angeordnet. Die Form und Größe dieser Kreisscheibe entspricht der Form und Größe der Sensorfläche des Lichtempfängers 27, bei dem es sich beispielsweise um einen Fototransistor handelt. Die Ausgangsleitungen 28 des Lichtempfängers 27 sind an ein elektronisches Steuergerät 29 angeschlossen, das eine Auswerteschaltung zur Auwertung der Signale des Lichtempfängers 27 und eine Zähleinrichtung zum Zählen der erkannten Tropfen enthält. Zwischen der Austrittsseite 26 des Lichtstrahl-Querschnittswandlers 23 und dem Lichtempfänger 27 befindet sich bei dem vorliegenden Ausführungsbeispiel ein Lichtfilter 30 zur Eleminierung von Fremdlichteinflüssen. Der Lichtsender 20 strahlt beispielsweise im Infrarotbereich.

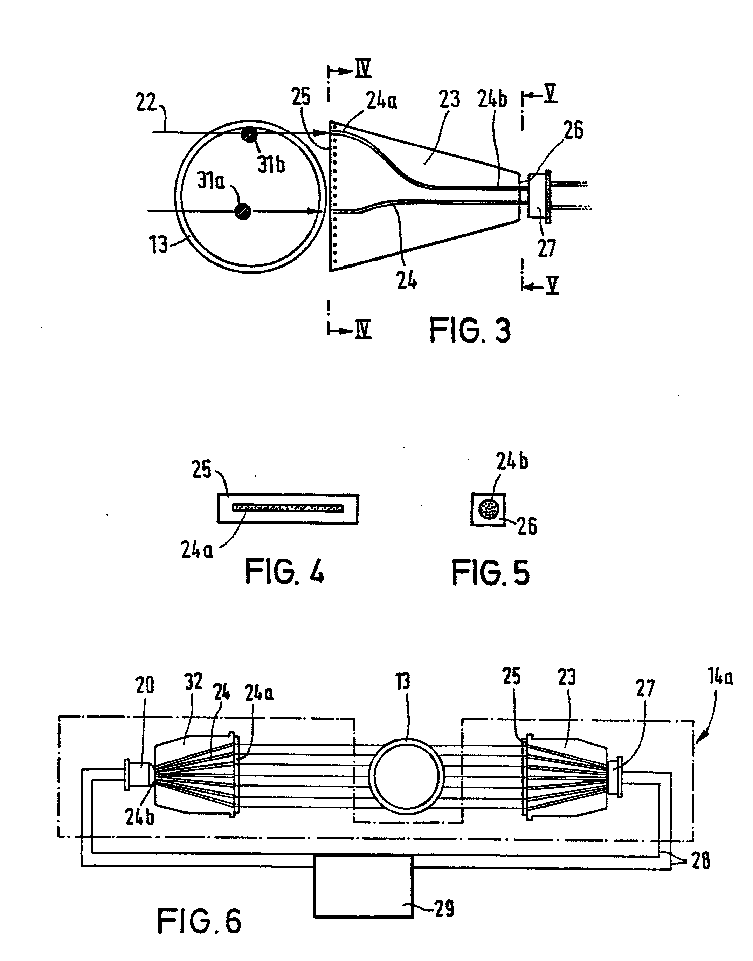

Während in Fig. 2 der Verlauf der Lichtleiter 24 lediglich schematisch dargestellt ist, ist dieser Verlauf in den Fign. 3 bis 5 genauer angegeben. Die Lichtleiter 24 sind innerhalb des Lichtstrahl-Querschnittswandlers 23 so verlegt, daß ihre Enden 24a an der Eintrittsseite 25 parallel zueinander verlaufen und rechtwinklig in die Eintrittsseite 25 münden. Auch auf der Austrittsseite 26 verlaufen die Enden 24b der Lichtleiter 24 parallel zueinander und sie münden rechtwinklig in die Austrittsseite 26, der die Sensorfläche des Lichtempfängers 27 gegenüberliegt.

Aus Fig. 3 erkennt man, daß ein Tropfen 31a, der in der Nähe der Mittelachse der Tropfkammer 13 herabfällt am Lichtempfänger 27 die gleiche Signaländerung hervorruft wie ein Tropfen 31b, der in der Nähe der Wand der Tropfkammer 13 herabfällt. Dies liegt daran, daß die Lichtstrahlen 22, die durch jeden der Tropfen 31a, 31b unterbrochen werden, senkrecht auf die Eintrittsfläche 25 und senkrecht auf die Sensorfläche des Lichtempfängers 27 treffen.

Die Eintrittsenden 24a der Lichtleiter 24 sind gemäß Fig. 4 in Form eines horizontalen länglichen Streifens angeordnet, sodaß sich ein Erfassungsbereich mit geringer vertikaler Erstreckung ergibt. An der Austrittsseite 26 sind die Enden 24b derselben Lichtleiter 24 gemäß Fig. 5 in Form einer Kreisscheibe angeordnet. Die Form und Größe der Kreisscheibe entspricht der Form und Größe der Sensorfläche des Lichtempfängers 27.

In dem Lichtstrahl-Querschnittswandler 23 sind die Lichtleiter 24 verklebt oder von einer Vergußmasse umgeben, sodaß sie relativ zueinander fixiert sind.

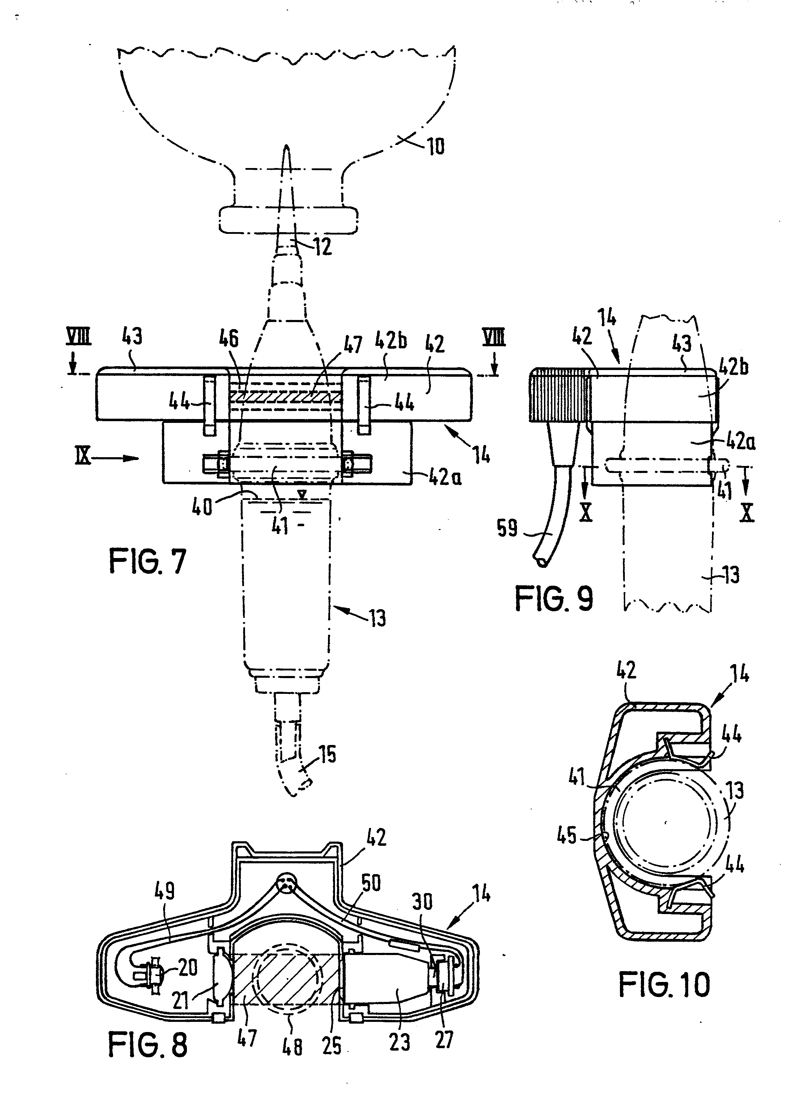

Der Tropfendetektor 14a nach Fig. 6 unterscheidet sich von dem zuvor beschriebenen Tropfendetektor 14, der schematisch in Fig. 2 dargestellt ist, nur dadurch, daß anstelle der Sammellinse 21 ein zweiter Lichtstrahl-Querschnittswandler 32 vorgesehen ist, der in gleicher Weise aufgebaut ist, wie der Lichtstrahl-Querschnittswandler 23, wobei jedoch der Lichtsender an demjenigen Ende 24b angeordnet ist, in dem das Lichtleiterfeld kreisförmig ist, während das langgestreckte Ende 24a der Tropfkammer 13 zugewandt ist. Auf diese Weise erzeugt der Lichtstrahl-Querschnittswandler 32 ein streifenförmiges Lichtband, dem der streifenförmige Empfangsbereich des Lichtstrahl-Querschnittswandlers 23 entspricht. Die parallel aus dem Ende 24a des Lichtstrahl-Querschnittswandlers 32 austretenden Lichtstrahlen treffen, wenn sie nicht an der Tropfkammer abgelenkt werden, genau auf die Empfangsfläche des Lichtstrahl-Querschnittswandlers 23, wobei das ausgesandte Licht vollständig empfangen wird, weil die lichtaussende Fläche und die lichtempfangende Fläche einander genau entsprechen und deckungsgleich sind. Fign. 7 bis 10 zeigen detaillierter die Konstruktion eines praktischen Ausführungsbeispiels des Tropfendetektors. Der Einstichdorn 12 der Tropfkammer 13 ist durch den Stopfen, der die Öffnung des Behälters 10 verschließt, hindurchgestochen. In der aus durchsichtigem Material bestehenden Tropfkammer tropft Infusionslösung herab. Im Innern der Tropfkammer 13 bildet sich ein Flüssigkeitspegel 40. Die Tropfkammer 13 ist aus zwei Gehäuseteilen hergestellt, die an ihrer Stoßstelle einen umlaufenden Ringwulst 41 bilden, in dem die Gehäuseteile miteinander verbunden sind. Dieser Ringwulst 41 wird dazu benutzt, das Gehäuse 42 des Tropfendetektors an der Tropfkammer 13 zu befestigen. Das Gehäuse 42 ist in Draufsicht U-förmig (Fign. 8 und 10) und es weist einen unteren Haltebereich 42a zum Festklemmen an der Tropfkammer 13 und einen oberen Detektorbereich 42b zur Aufnahme der optischen Elemente des Tropfendetektors auf. Der untere und der obere Gehäusebereich 42a, 42b sind fest miteinander verbunden. Der obere Gehäusebereich 42b ist an seiner Oberseite offen. Die Oberseite ist durch einen abnehmbaren Deckel 43 verschlossen, der durch Laschen 44 am oberen Gehäusebereich 42b verriegelt ist.

Der untere Gehäusebereich 42a ist im Bereich der Ausnehmung zum seitlichen Einschieben der Tropfkammer 13 mit einer horizontalen Nut 45 versehen, in die der Ringwulst 41 der Tropfkammer 13 einschiebbar ist (Fig. 10). Durch die Nut 45 wird das Gehäuse 42 gegen Längsverschiebungen in bezug auf die Tropfkammer 13 gesichert. An den beiden Enden der Nut 45 sind am Gehäuse 42 Blattfedern 44 befestigt, die die aus der Nut 45 herausragenden Bereiche des Ringwulstes 41 umgreifen und die Tropfkammer 13 im Gehäuse 42 sichern. Die Enden der Blattfedern 44 sind nach außen gebogen, um die Tropfkammer 13 unter Aufspreizung der Blattfedern 44 in das Gehäuse 42 seitlich einschieben zu können.

Der Lichtsender 20 und die Linse 21 sind gemäß Fig. 8 im linken Teil des Gehäuses 42 angeordnet, während der Lichtstrahl-Querschnittswandler 23, der Lichtfilter 30 und der Lichtempfänger 27 im rechten Gehäuseteil angeordnet sind. Zwischen den beiden Gehäuseteilen befinden sich der seitliche offene Bereich zum Einführen der Tropfkammer 13.

Die Linse 21 erzeugt aus dem Licht des Lichtsenders 20 ein durch den oberen Bereich der Tropfkammer 13 quer hindurchgehendes Ausleuchtungsfeld 46, dessen vertikale Erstreckung größer ist als die vertikale Erstreckung des - scheibenförmigen Erfassungsbereichs 47 des Lichtstrahl-Querschnittswandlers 23. Der Erfassungsbereich 47 ist in den Fign. 7 und 8 schraffiert dargestellt. Außerdem ist in Fig. 8 die Innenkontur 48 der Tropfkammer 13 in Höhe des Erfassungsbereichs 47 abgebildet. Man erkennt, daß die Breite des Erfassungsbereichs 47 etwa der Innenweite der Tropfkammer an dieser Stelle entspricht und daß die vertikale Erstreckung des Erfassungsbereichs 47 viel kleiner ist als dessen horizontale Breite.

In Fig. 8 sind ferner die zum Lichtsender 20 führenden elektrischen Versorgungsleitungen 49 und die von den Lichtempfänger 27 abführenden Signalleitungen 50 erkennbar. Die Leitungen 49 und 50 führen zu einem Kabel 51 (Fig. 9) , das an das in Fig. 2 dargestellte Steuergerät 29 angeschlossen werden kann.

**Ansprüche**

1. Tropfendetektor, mit mindestens einem Lichtsender und einem Lichtempfänger, die an entgegengesetzten Seiten einer lichtdurchlässigen Tropfkammer positionierbar sind,

dadurch gekennzeichnet, daß im Strahlenweg vor dem Lichtempfänger (27) ein Lichtstrahl-Querschnittswandler (23) angeordnet ist, der einen der im wesentlichen der lichten Weite der Tropfkammer (13) entsprechenden Erfassungsbereich aufweist und die aus dem Erfassungsbereich einfallende Strahlung in ein Strahlenbündel umsetzt, das die lichtempfindliche Sensorfläche des Lichtempfängers (27) im wesentlichen auffüllt.

2. Tropfendetektor nach Anspruch 1, dadurch gekennzeichnet, daß der Lichtstrahl-Querschnittswandler (23) aus einem Bündel von Lichtleitern (24) besteht, dessen Querschnittsform an der Eintrittsseite (25) von der Querschnittsform an der Austrittsseite (26) abweicht.

3. Tropfendetektor nach Anspruch 2, dadurch gekennzeichnet, daß die Enden der Lichtleiter (24) an der Eintrittsseite (25) des Bündels parallel zueinander verlaufen.

4. Tropfendetektor nach Anspruch 2 oder 3, dadurch gekennzeichnet, daß die Enden (24b) der Lichtleiter an der Austrittsseite (26) des Bündles parallel zueinander verlaufen.

5. Tropfendetektor nach einem der Ansprüche 1 bis 4, dadurch gekennzeichnet, daß der Erfassungsbereich die Form einer Scheibe hat, die quer durch die Tropfkammer (13) hindurchgeht und rechtwinklig zur Tropfkammerachse verläuft und deren Dicke wesentlich kleiner ist, als der Durchmesser der Tropfkammer (13).

6. Tropfendetektor nach einem der Ansprüche 2 bis 5, dadurch gekennzeichnet, daß die Enden - (24a, 24b) der Lichtleiter (24) an der Eintrittsseite - (25) des Bündels in Form eines länglichen Streifens und an der Austrittsseite (26) in Form eines Kreises angeordnet sind.

7. Tropfendetektor nach einem der Ansprüche 1 bis 6, dadurch gekennzeichnet, daß der Lichtsender (20) ein Infrarotsender ist und daß zwischen der Tropfkammer (13) und dem Lichtempfänger - (27) ein Lichtfilter (30) angeordnet ist.

8. Tropfendetektor nach einem der Ansprüche 1 bis 7, dadurch gekennzeichnet, daß hinter dem Lichtsender (20) einer Einrichtung (21) angeordnet ist, die ein paralleles Strahlenbündel liefert, dessen Breite im wesentlichen der Weite der Tropfkammer (13) entspricht.

9. Tropfendetektor nach Anspruch 8, dadurch gekennzeichnet, daß hinter dem Lichtsender (20) ein zweiter Lichtstrahl-Querschnittswandler (32) angeordnet ist, dessen lichtaussendende Fläche in Form und Größe im wesentlichen der lichtempfangenden Fläche des ersten Lichtstrahl-Querschnittswandlers (23) entspricht und auf diese gerichtet ist.

FIG. 1

FIG. 2

FIG. 3

FIG. 4

FIG. 5

FIG. 6

0 209 659

FIG. 7

FIG. 9

FIG. 8

FIG. 10